(19)

Europäisches
Patentamt
European
Patent Office
Office européen
des brevets

(11) **EP 4 336 460 A1**

(12) **EUROPEAN PATENT APPLICATION**

(43) Date of publication:
**13.03.2024   Bulletin 2024/11**

(21) Application number: **22194697.3**

(22) Date of filing: **08.09.2022**

(51) International Patent Classification (IPC):
**G06T 19/20** *(2011.01)*

(52) Cooperative Patent Classification (CPC):
**G06T 19/20;** G06T 2210/41; G06T 2219/2021

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TheraPanacea
75004 Paris (FR)**

(72) Inventors:
• **PARAGIOS, Nikos**
  **75004 Paris (FR)**
• **LEROY, Amaury**
  **75004 Paris (FR)**

(74) Representative: **Icosa
83 avenue Denfert-Rochereau
75014 Paris (FR)**

(54) **METHOD AND DEVICE FOR GENERATING A COMPLETE 3D REPRESENTATION OF AN OBJECT FROM A PARTIAL SIGNAL**

(57)   The present invention relates to a method for generating an adjusted (or augmented) 3D representation of an object based on a reference 3D source representation compliant with a source imaging modality and a plurality of reference target images compliant with a target imaging modality and establishing meaningful anatomical correspondences between the 3D object representation and the 2D partial/sparse view of the object, comprising: obtaining, from reference target images, corresponding source images compliant with the source imaging modality; obtaining a sparse 3D source representation whose 2D sections correspond to the obtained source images; determining, from the reference 3D source representation and the sparse 3D source representation, a deformation field to be applied to voxels of the reference 3D source representation so as to register 2D sections of the reference 3D source representation with corresponding 2D sections of the sparse 3D source representation; obtaining the adjusted 3D representation of the object by applying the deformation field to the 3D reference source representation.

```
┌─────────────────────────┐
│  Receiving reference    │
│  3D source              │ ─── 615
│  representation         │
└─────────────────────────┘
            │
            ▼
┌─────────────────────────┐
│  Receiving operational  │
│  2D target images       │ ─── 625
└─────────────────────────┘
            │
            ▼
┌─────────────────────────┐
│  Receiving operational  │
│  trained model          │ ─── 635
└─────────────────────────┘
            │
            ▼
┌─────────────────────────┐
│  Determining adjusted   │
│  3D representation      │ ─── 645
└─────────────────────────┘
```

**FIGURE 6**

EP 4 336 460 A1

**Description**

**FIELD OF THE INVENTION**

The invention relates to the field of image synthesis and image processing, and more specifically to a method and a device for generating an augmented (i.e., adjusted) 3D representation of an object, for example an area of a human body.

**BACKGROUND OF THE INVENTION**

In the context of interventional procedures, doctors use medical imaging data to perform a diagnostic or therapeutic or a treatment response evaluation act. For example, interventional radiology combines a radiological imaging technique (using X-rays) with an invasive procedure for diagnostic and/or therapeutic purposes. The intervention is guided and controlled by the radiological image. This is also the case in the domain of radiotherapy where radiological planning data are combined with online imaging data to deliver, adapt and guide treatment.

Usually, a first series of images of a patient's body area is taken before the intervention, for example approximately one week before the intervention. This first series of images, called "planning data" or "planning images", is used by the doctor to prepare the intervention, in particular by visualizing the body area on which he must intervene and by locating the diseased tissues as well as determine areas and tissues that are to be preserved. During the intervention, the doctor is guided by a second series of images, called "interventional images" or "interventional data", of the patient's body area obtained in "near" real time and are used to guide the process.

Planning images should be as accurate as possible, and are generally high or very high resolution images, eventually 3D images, obtained using computationally expensive image processing methods. Due to the absence of acquisition constraints, these data are usually multi-modal exploiting complementarity of information across imaging modalities (computed tomography, magnetic resonance imaging, positron emission tomography, etc.) and associated with the highest possible level of resolution. Planning images may for example be obtained with a 3D computed tomography scan (or "3D CT scan") technique. Conversely, interventional images must be obtained quickly and regularly during the intervention, to have real-time information on the area of the body to be treated. For this reason, interventional images are usually lower resolution images, and could be generally multiple planes 2D images. For example, interventional images are obtained with a 2D Magnetic Resonance (MR) Imaging technique.

Other types of images may be used. For example, in the context of guided breast or prostate biopsies, the planning images may be CT and/or MR images and the interventional images may be ultrasound images. In the context of cardiac or brain surgeries, the planning images may be CT and/or MR images and the interventional images may be angiography images. In the context of radiotherapy (RT), the planning images may be CT images and the interventional images may be X-rays and/or 2D-MR images. In the context of radiation therapy planning data can be 3D CT or MR images and treatment guidance data could be sparse 2D MR acquisitions or low resolution 3D MR sequences.

During the intervention, the physician has to make the link between the interventional images that he receives in real time and the planning images (i.e. to "map" the two series of images), to better adapt the treatment or the diagnosis. Indeed, mapping 3D planning data into the 2D interventional images enables the overlay of each image's specific information and is thus essential for successful treatment. However, performing a mapping of the 2D images obtained during the intervention on the 3D planning images is not always easy, in particular due to the position and movement of the patient during the intervention, but also to the difference in resolution between images. The time interval between the 3D planning sequence and the 2D treatment implementation could lead to important anatomical changes associated either with tissue collapse due to the surgical operation or to normal physiological changes of the human body.

In this context, methods have emerged in recent years to register a second series of planning images (usually low resolution and/or 2D images obtained during an interventional procedure) with a first series of planning images (usually high resolution and/or 3D images previously obtained). When the planning images represent 3D volumes and the interventional images are 2D frames, these methods are called "Slice-to-Volume (SV) registration". Image registration consists in aligning and combining data to obtain one unique coordinate system. SV registration consists in determining a slice from a given 3D volume that corresponds to an input 2D image. For example, an SV registration method makes it possible to determine to which slice of the 3D volume resulting from the planning data corresponds an interventional 2D image. When several slices of the 3D volume corresponding to several input 2D images are determined, the method is called "Multi-Slice-to-Volume (MSV) registration".

Some popular clinical settings in this context of Slice-to-Volume registration refer to ultrasound towards CT/MR (guided breast or prostate biopsies), angiography to CT/MR for cardiac and brain surgeries, or X-rays/2D MR to CT for radiotherapy (RT). Matching radiology and digital pathology is another domain where the same challenges are to be addressed due to the partial correspondences between the recovered specimen and prior 3D imaging acquisition.

In this respect, one has to face three main challenges: (i) anatomical changes and tissue deformation, (ii) sparse, partial-view and low-quality data during treatment implementation due to acquisition time and nature of signal limitations, and (iii) difference on the nature/modality of images to be aligned/registered.

Early literature to solve these challenges is mostly focused on solving the registration problem, with the objective of determining a mapping between the interventional data and the planning 3D images by covering mostly plane selection and in-plane deformations. Traditional Deformable Image Registration (DIR) methods iteratively align two volumes through optimization of an energy function. Recently, learning-based methods have spread out due to impressive performance and the ability to infer transformation parameters for any input volume pair from a unique model, first in a supervised setting, and more and more without labels.

However, the existing solutions have drawbacks, because they require complex calculations on too many data which make them difficult to use in an interventional context, are not scalable enough with respect to the sparsity and the nature of interventional signal, fail to deal with severe changes in the nature of the images to be registered, require excessive computing resources and computational time that prohibits their use in a real clinical setting or do not give sufficiently accurate results.

## SUMMARY OF THE INVENTION

The present invention aims to adjust a reference 3D representation of an object (e.g. an area of the human body on which a doctor must intervene) compliant with an imaging modality, called "source imaging modality", so that the adjusted 3D representation coincides at least partially with 2D interventional images compliant with another imaging modality, called "target imaging modality". In other words, the 3D representation compliant with the source imaging modality is registered onto the 2D images compliant with the target imaging modality. For example, the source imaging modality may be a computed tomography scan and the reference 3D representation compliant with the source imaging modality may be a planning 3D CT scan. The target imaging modality may be Magnetic Resonance Imaging and the 2D interventional images compliant with the target imaging modality may be interventional 2D MR images. Sections of the adjusted 3D representation are therefore registered with the 2D interventional images, which allows the doctor to have a 3D representation of the area of the human body on which he must intervene which is as faithful as possible to the real area at the time of the intervention.

To achieve this aim, the invention proposes a compositional solution that relies simultaneously on two learning models: one concerns the automatic translation from one imaging modality to another, and the other the elastic registration of 3D representations to the partial 2D interventional images. The translation model creates a bijective model able to transform interventional 2D images compliant with the target imaging modality into 2D images into similar 2D images compliant with the source imaging modality. These similar 2D images compliant with the source imaging modality can therefore be used to build a 3D sparse representation of the object compliant with the source imaging modality. The 3D reference representation may then be deformed and adjusted to the obtained 3D sparse representation via the registration model. Joint learning of the two learning blocks may advantageously performed in a completely unsupervised way, and allows to obtain a more accurate adjusted representation of the object than existing methods, because each learning block benefits from the other. In particular, registration is done in a faster and more precise way because the two input representations are compliant with the same imaging modality. The translation block benefits from a backpropagation of the loss function of the registration block, which allows faster convergence and a more efficient overall learning model.

A first aspect of the invention therefore relates to a method implemented by computer for training a machine learning architecture for generating an adjusted 3D representation of an object based on a reference 3D representation of said object compliant with a source imaging modality and a plurality of images of at least part of said object compliant with a target imaging modality, so that 2D sections of the adjusted 3D representation are at least partially registered with images of the plurality of images, the machine learning architecture comprising a first machine learning architecture and a second machine learning architecture. The method may comprise:

- receiving training target images compliant with the target imaging modality and 3D training source representations compliant with the source imaging modality;
- obtaining training source images corresponding to 2D sections of the received 3D training source representations; and
- jointly training the first machine learning architecture and the second machine learning architecture to obtain:

  o a first learned model associated with the first machine learning architecture, said first learned model being adapted to generate, from an input target image compliant with the target imaging modality, a simulated source image compliant with the source imaging modality respectively associated with the input target image; and

  o a second learned model associated with the second machine learning architecture, said second learned model being adapted to generate, from a sparse 3D representation compliant with the source imaging modality and an original 3D representation compliant with the source imaging modality, a deformation field to be applied to voxels of the original 3D representation so as to obtain an adjusted 3D representation whose 2D sections are at least partially registered with 2D sections of the sparse 3D representation.

The first machine learning architecture may be trained based on first set of training data comprising the training target images and the training source images.

The second machine learning architecture may be trained based on a second set of training data comprising the received 3D training source representations and sparse 3D representations compliant with the source imaging modality. Said sparse 3D representations may be obtained by:

- obtaining, from at least part of the training target images, simulated source images compliant with the target imaging modality through the first machine learning architecture; and
- constructing the sparse 3D representations so that sections of said sparse 3D representations correspond to said simulated source images.

By "object", it is meant any element whose representation is obtained using an imaging technique. For example, the object may be a human body part.

The term "imaging modality" refers to the imaging technique used for obtaining 3D or 2D images. For example, the imaging modality may designate the imaging technology (for example, in the field of medical imaging, an imaging technology among: x-ray imaging, scanner, scintigraphy, positron emission tomography, ultrasound, magnetic resonance imaging, etc.), but also specific characteristics of the imaging technique used (e.g. resolution).

By "image or representation compliant with the imaging modality", it is meant an image or a representation that have been obtained or synthetized according to the same imaging acquisition principles or that could have been obtained by using said imaging modality. In other words, the wording also includes images or representations that have been generated by a processing technique and which simulated images or representations obtained by the imaging modality.

By "source imaging modality", it is meant the imaging modality associated with the 3D representation to register. The wording "target imaging modality" refers to an imaging modality distinct from the target imaging modality, and designates the imaging modality associated with the 2D images onto which the 3D representation shall be registered. Terms "target" and "source" are commonly used in the field of image registration, but they could be replaced by any other denominations.

By "adjusted 3D representation", it is meant the input 3D representation of the object (i.e. the "reference 3D representation") is deformed to be registered on a set of images (the "plurality of images"). For example, the reference 3D representation may be a planning 3D CT scan of a body area of a subject and the plurality of images may be a set of interventional MR images or angiography images or con beam CT images. In such cases, registration is necessary to cope the deformation of the subject, which may be due for example to breathing, movements, anatomical changes, ...

The challenges to be met here are on the one hand the fact that the 3D representation must be registered from a plurality of 2D images, and on the other hand the fact that the 3D representation and the set of 2D images do not come from the same imaging modality and the underlying signal differences make the establishment of meaningful anatomical correspondences challenging.

By "2D section of a 3D representation", it is meant a 2D representation of a section (or a "slice") of the 3D representation. For example, the 3D representation may comprise a voxel grid along 3 axes (x,y,z). A 3D section of the 3D representation may correspond to the subset of voxels (x,y) when one of the coordinate (z) is fixed. The corresponding 2D section may then be defined as the 2D projection of the 3D section on a plane (x,y). For example, a respective pixel (x,y) may be associated with each voxel (x,y) of the 3D section and its value may be set to the value of the corresponding voxel.

In the context of CT scan, the sections may advantageously cross sections, but the sections may be performed along any axis (longitudinal, sagittal, or other random axis orientation of interest).

By "sparse 3D representation" it is meant a 3D representation of which some sections correspond to images of the object (non-empty sections), and other sections are empty or filled with predefined values (for example, null values). This sparse 3D representation has the same size (along the 3 axes x, y, z) as the reference 3D representation. The non-empty sections may be filled with corresponding 2D images (e.g., the simulated source images), while the empty sections may be filled with zero values. To fill the non-empty sections, it is possible, for example, to associate to each voxel of a non-empty section a value corresponding to a respective pixel of a 2D image.

Therefore, the sparse 3D representations of the second said of training data are constructed so that sections of said sparse 3D representations are filled according to the pixel values of the simulated source images.

By "deformation field", also called "displacement field", it is meant a set of data (voxel-wise 3D vector displacement) characterizing a transformation to be applied to a 3D representation to perform registration. This deformation field may advantageously concatenate two components: a first component corresponding to a linear (rigid, similarity, affine, etc.) transformation, and a second component corresponding to a non-rigid (or "elastic") transformation.

The first learned model generates, from one image corresponding to a given imaging modality, a corresponding image that "synthetizes" the image that would have been obtained of the same object by using another imaging acquisition principle than the one that have been used. In other words, the overall appearance (including edges) is the same between the input image and the simulated image, but the simulated image "looks like" an image obtained by the other imaging modality.

The second learned model registers one 3D representation with another 3D representation. In the context of the present invention, this other 3D representation is sparse. Therefore, this registration is performed on non-empty (or non-zero) intersections between the deformed 3D planning representation and the interventional 2D sparse signal in terms of image correspondences and is constrained to be regular and smooth on the whole 3D domain. More specifically, as detailed hereinafter, registration is performed on all sections of the sparse representation (empty and non-empty), but to assess the quality of registration (measurable via the loss function associated with the second ML architecture), only the non-empty sections are considered. In addition, a regularization component of the loss function may be added, to impose a continuous deformation field, and thus a smoothing between the successive sections. Therefore, two neighbor voxels (according to any direction x, y or z) must have "close" displacements applied to them. This makes it possible to respect a certain similarity between the empty sections and the non-empty sections, and to obtain a coherent deformation field, even for the empty voxels.

According to the invention, the machine learning (ML) architecture comprises a first ML architecture for performing the translation from one imaging modality to another, and a second ML architecture for performing the registration between two 3D representations. The first and the second ML architectures are jointly trained. Therefore, each ML architecture benefits from the other. More specifically, the second ML architecture takes advantage of the fact that the input 3D representations correspond to the same imaging modality, which improves the quality of the registration. The first ML architecture takes advantage of the backpropagation of the error associated with the second ML architecture, which improves performance of translation from one imaging modality to another. The two training architectures they may be either trained independently, used as pre-trained models and re-trained jointly or being trained simultaneously through an end-to-end principle.

In one or several embodiments, the first machine learning architecture may be associated with a first loss and the second machine learning architecture may be associated with a second loss. The joint training of the first machine learning architecture and the second machine learning architecture may comprise a joint minimization of the first loss and the second loss weighed according to the importance of the two tasks

In one or several embodiments, the first machine learning architecture and/or the second machine learning architecture may be trained in an unsupervised manner.

In one or several embodiments, each of the 3D training source representations may represent a part of a body of a respective subject among a plurality of subjects. The training target images may comprise partial or full sets of training target images, each set of training target images being associated with a respective subject of the plurality of subjects and representing successive sections of the part of the body of said respective subject.

In other words, for each subject of a plurality of subjects, there are one 3D training source representation and a set of training target images. These representation and images are used for training the ML architecture according to the above method.

In one or several embodiments, the first machine learning architecture may be adapted to synthetize, from an image compliant with one imaging modality among the source imaging modality and the target imaging modality, a corresponding image compliant with the other imaging modality. The first machine learning architecture may further be trained based on pairs of images, each pair of images comprising:

- a training image compliant with one imaging modality among the target imaging modality and the source imaging modality, said training image being one of the training source images and the training target images; and
- a corresponding new image compliant with the same imaging modality and obtained from the training image of the pair through the first machine learning architecture.

For example, for each pair of images, the new image may be obtained by:

- obtaining, from the training image of said pair, a corresponding intermediate image compliant with the other imaging modality through the first machine learning architecture; and
- obtaining, from the obtained intermediate image, the new image through the first machine learning architecture.

In one or several embodiments, the first loss may be function of:

- a first term representative of a similarity between a training image among the training source images and the training target images, said training image being compliant with one imaging modality among the target imaging modality and the source imaging modality, and a corresponding image compliant with the other imaging modality and generated from said training image through the first machine learning architecture; and

- a second term representative of the similarity between the images of a pair of images.

In one or several embodiments, the target imaging modality may be magnetic resonance imaging and the source imaging modality may be computed tomography imaging.

In one or several embodiments, the first machine learning architecture may comprise a cycle generative adversarial network, GAN.

In one or several embodiments, the second machine learning architecture may comprise a convolutional neural network, CNN.

Another aspect of the invention relates to a method implemented by computer for generating an adjusted 3D representation of an object based on a reference 3D source representation of said object compliant with a target imaging modality and a plurality of reference target images of at least part of said object compliant with a target imaging modality. The method may comprise:

- obtaining a plurality of synthetized source images compliant with the source imaging modality and respectively corresponding to the plurality of reference target images, by applying the first learned model to reference target images;
- obtaining a sparse 3D source representation whose 2D sections correspond to simulated source images among the plurality of simulated source images;
- determining, by applying the second learned model to the reference 3D source representation and the sparse 3D source representation, a deformation field to be applied to voxels of the reference 3D source representation so as to at least partially align 2D sections of the reference 3D source representation with corresponding 2D sections of the sparse 3D source representation; and
- obtaining the adjusted 3D representation of the object by applying the determined deformation field to the 3D reference source representation.

In one or several embodiments, the reference target images may represent successive sections of at least part of the object. For example, the reference target images may be successive images of a body area along a longitudinal axis.

Yet another aspect of the invention relates to a device comprising a processor configured to carry out any of the above methods. The device may also comprise an input interface to receive data and an output interface to output data.

Yet another aspect of the invention relates to a computer program product comprising instructions which, when the program is executed by a computer, cause the computer to carry out any of the above methods.

## BRIEF DESCRIPTION OF THE DRAWINGS

The present invention is illustrated by way of example, and not by way of limitation, by the figures in which:

**Figure 1** is an example of a flow-chart representing a method for training a machine learning architecture for generating an adjusted 3D representation of an object according to embodiments of the invention;

**Figure 2** is a block diagram representing an example of a device for training a machine learning architecture for generating an adjusted 3D representation of an object according to embodiments of the invention;

**Figure 3** is an example of a flow-chart representing a method for training a first machine learning architecture for generating, from an image compliant with an imaging modality, an image compliant with another imaging modality according to embodiments of the invention;

**Figure 4** represents an example of the first machine learning architecture according to embodiments of the invention;

**Figure 5** represents an example of a second machine learning architecture for performing registration between two 3D representations according to embodiments of the invention;

**Figure 6** is an example of a flow-chart representing a method for generating an adjusted 3D representation of an object according to embodiments of the invention;

**Figure 7** is a block diagram representing an example of a device for generating an adjusted 3D representation of an object according to embodiments of the invention;

**Figure 8** illustrates a device for training a machine learning architecture for generating an adjusted 3D representation of an object and/or for generating an adjusted 3D representation of an object according to embodiments of the invention.

## DETAILED DESCRIPTION

Expressions such as "comprise", "include", "incorporate", "contain", "is" and "have" are to be construed in a non-exclusive manner when interpreting the description and its associated claims, namely construed to allow for other items or components which are not explicitly defined also to be present.

The terms "adapted", "augmented" and "configured" are used in the present disclosure as broadly encompassing initial configuration, later adaptation or complementation of the present device, or any combination thereof alike, whether effected through material or software means (including firmware).

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processorreadable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

Without loss of generality, it is considered in the following that the source imaging modality is Computed Tomography (CT) scan imaging and the target imaging modality is Magnetic Resonance (MR) imaging. The reference 3D representations are therefore 3D CT scan volumes representing a whole area of a human body of a subject and the plurality of images compliant with the target imaging modality are 2D MR images of random / arbitrary views partially representing said area of the human body. By "partially", it is meant that each of the MR images represent area spaced sufficiently apart that a 3D reconstruction of the subject's body area is not possible from the images as they are. For example, MR images represent 2D cross sections of the area spaced several tens of millimeters apart, e.g. 1 to 2 centimeters. Possibly, the MR images themselves only represent a part of the corresponding cross-section of the associated 3D representation. For example, the 3D CT scan can cover a complete area of the body, e.g. the whole abdomen, and the 2D MR images can represent only part of the abdomen, e.g. the stomach. Of course, the invention extends to other imaging modalities.

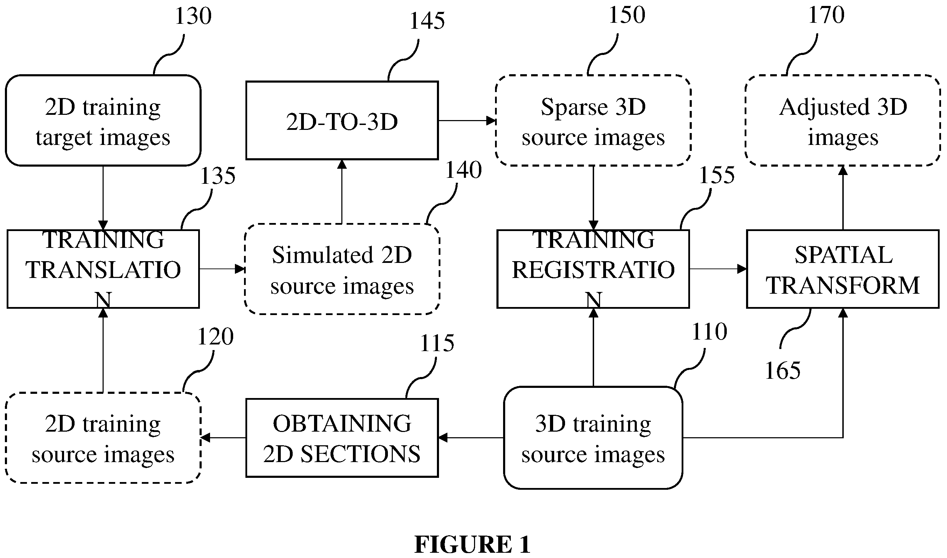

**Figure 1** is an example of a flow-chart representing a method for training a machine learning architecture for generating an adjusted 3D representation of an object according to embodiments of the invention.

In Figure 1, training data is represented by rectangles with rounded corners in a solid line. Conversely, data obtained from processing is represented in rectangles with rounded corners and in dashes. The training data comprises a set of 3D training representations compliant with the source modality 110 (e.g. 3D CT scans of an area obtained from plurality of subjects) and a set of pluralities of 2D training images compliant with the target modality 130 (e.g. a set of 2D MR images, the set of 2D MR images comprising several pluralities of 2D RM images, each plurality of 2D RM images being obtained from a respective plurality of subjects and corresponding to cross sections of at least part of the same area as the 3D CT scans). For example, training data may have been obtained from a plurality of subjects and comprise, for each subject: a 3D CT scan of an area of the subject body and a plurality of 2D RM images of at least part of the area of the subject body.

First, the 3D CT scans 110 may be processed (step 115) to obtain, for each 3D CT scan 110, a corresponding group of 2D CT images 120. For example, for a given 3D CT scan 110 of a subject, the corresponding group of 2D CT images 120 may comprise successive sections (e.g. cross sections) of said 3D CT scan. For example, these sections may be obtained randomly from the 3D CT scan 110. For instance, the 3D CT scan 110 may comprise a grid of voxels with respective dimensions $X_{CT}$, $Y_{CT}$, $Z_{CT}$ along three directions (x, y, z). To obtain one 2D CT image 120, it is possible to randomly draw an integer k between 1 and $Z_{CT}$, to obtain the section of voxels corresponding to z = k and to define the 2D image CT as a 2D grid of pixels having the same values as the obtained section of voxels. Other processing techniques are possible. Such techniques are known by the person skilled in the art and are not further detailed. Advantageously, for each subject, the number of 2D CT images 120 extracted from the 3D CT scan 110 is equal to the number of 2D MR images 130.

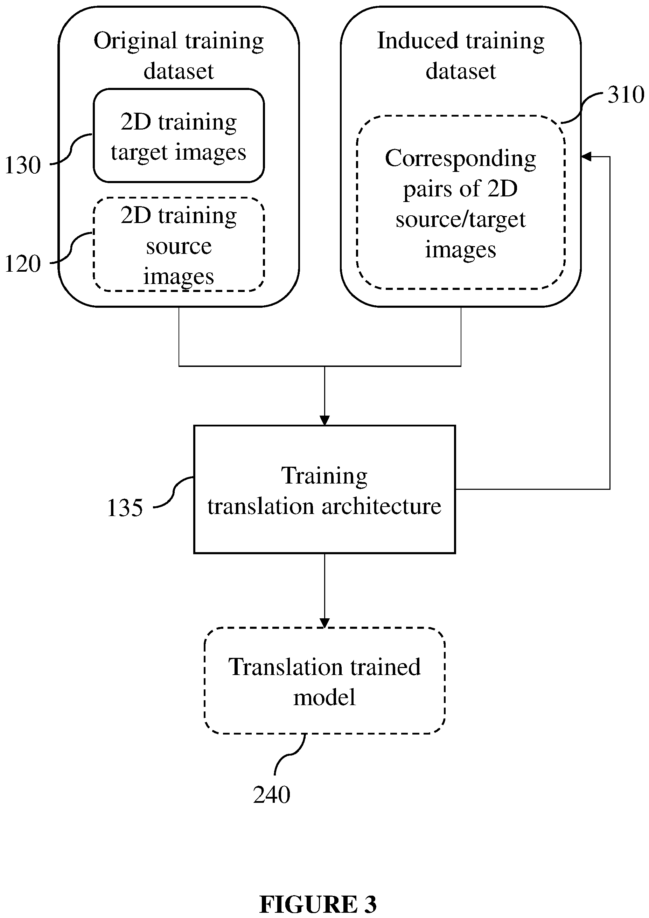

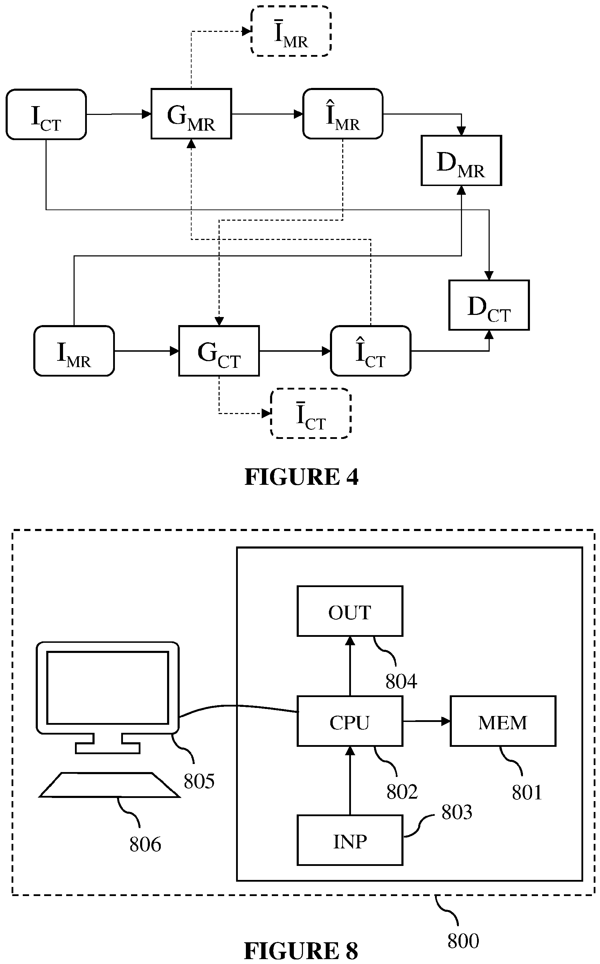

The training 2D MR images 130 and the obtained 2D CT images 120 are used to train (step 135) a first machine learning (ML) architecture so as to obtain a first learned model adapted to perform a translation from the target imaging modality to the source imaging modality (e.g. MR-to-CT translation), i.e. to generate, from an image compliant with the target imaging modality, a corresponding image compliant with the source imaging modality. The first learned model allows to "transform" a 2D MR image into a corresponding 2D CT scan image, called "2D pseudo-CT image". In other words, the obtained 2D pseudo-CT image simulates the image one would have obtained using a CT scan imaging technique instead of the MR imaging technique. The training (step 135) of the first ML architecture is further detailed below, with reference to Figures 3 and 4.

The first ML architecture outputs a set of 2D pseudo-CT images 140 respectively associated with the set of training 2D MR images 130. These 2D pseudo-CT images 140 are then processed (step 145) to obtain sparse 3D pseudo-CT representations 145.

In other words, at step 145, for each plurality of 2D pseudo-CT images 140 corresponding to the same subject, a corresponding 3D pseudo-CT representation 150 is constructed. The 2D pseudo-CT images 140 correspond to sections (e.g. cross sections) of the constructed 3D pseudo-CT representation 150. The 3D pseudo-CT representation 150 is therefore "sparse", since volumes between two sections are empty. In embodiments, these empty volumes may be filled by zero values. For example, the 3D pseudo-CT representation 150 may be a regular grid of voxels in 3D space. The values of the voxels of one section of the 3D pseudo-CT representation 150 may be set according to the values of the pixels of the corresponding 2D pseudo-CT image 140. The values of the voxels of the 3D pseudo-CT representation 150 that do not correspond to a 2D pseudo-CT image 140 may be set to zero or to any other reference value.

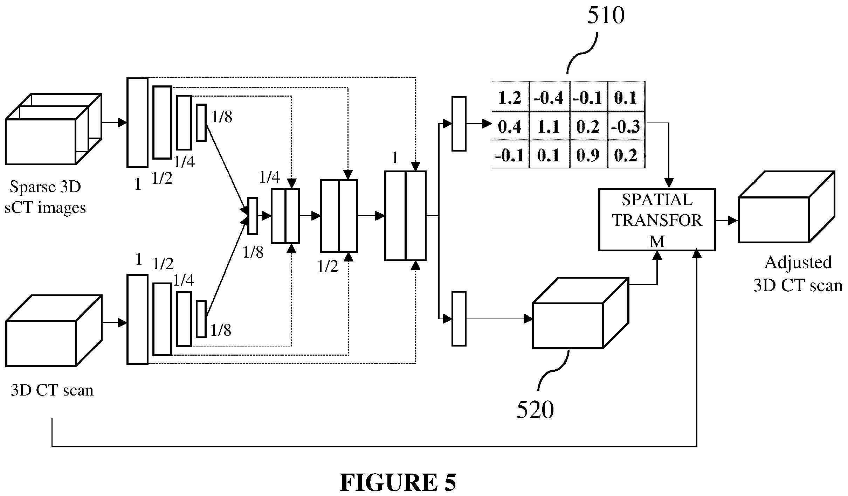

The obtained 3D pseudo-CT representations 150 are used, together with the original 3D CT scans 110, to train a second ML architecture (step 155) so as to obtain a second learned model adapted to perform a registration between an original 3D CT scan 110 and a corresponding 3D pseudo-CT representation 150, i.e. to determine a transformation (e.g. a deformation field) to apply to the original 3D CT scan 110 to align certain points of interest (e.g. points corresponding to edges of non-empty sections, i.e. sections that do not correspond to empty sections of the associated 3D pseudo-CT representation 150) with the corresponding points of the 3D pseudo-CT representation 150. The training (step 155) of the second ML architecture is further detailed below, with reference to Figure 5.

The transformation determined at step 155 is then applied (step 165) to the original 3D CT scans 110 to obtain transformed 3D CT scans 170 at least partially registered with corresponding 3D pseudo-CT representations 150.

To sum up, training data 120 and 130 used to train the first ML architecture comprise images obtained from a plurality of subjects. More specifically, for each subject, training data comprises 2D RM images 130 and 2D CT scan images 120 (obtained by slicing the 3D CT scan 110 of the subject). From the 2D RM images 130 of one subject, corresponding 2D pseudo-CT images 140 are obtained as outputs of the first ML architecture (step 140). These 2D pseudo-CT images 140 simulate images that would have been obtained instead of the 2D RM images 130 using CT scan imaging instead of RM imaging on the subject. These 2D pseudo-CT images 140 are then used to construct a 3D pseudo-CT representation 150 of which certain sections correspond to the 2D pseudo-CT images 140. This 3D pseudo-CT representation 150 is sparse, since only a few sections are available. Then, the 3D CT scan 110 of the subject is deformed, or "adjusted", (steps 155 and 165) to be at least partially registered with the sparse 3D pseudo-CT representation 150 obtained for the subject.

It is noted that the first and the second ML architectures are not trained independently, but jointly. In other words, the first ML architecture is associated with a first loss, the second ML architecture is associated with a second loss, and the first loss and the second loss are jointly minimized to train the first and the second ML architectures. Training data comprises pairs of data (3D CT scan 110 and set of 2D RM images 130) obtained from a plurality of subjects, and for each subject, a 3D pseudo-CT scan 150 is generated by the first ML architecture from the respective set of 2D RM images 130, and this 3D pseudo-CT scan 150 is forwarded to the second ML architecture to warp the original 3D CT scan 110.

In other words, if the first ML architecture (dedicated to translation) is associated with a first loss $L_{trans}$ and the second ML architecture (dedicated to registration) is associates with a second loss $L_{reg}$, the total loss $L_{total}$ for the whole ML architecture (translation and registration) may be a linear combination of $L_{trans}$ and $L_{reg}$. For example, $L_{total} = L_{trans} + L_{reg}$. The first and the second loss are jointly minimized during training of the complete ML architecture, to minimize $L_{total}$.

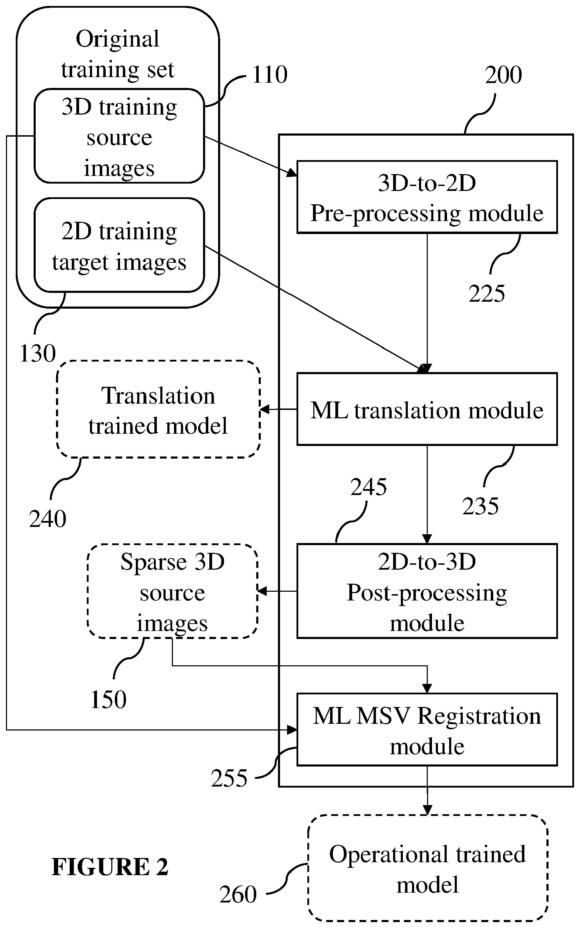

**Figure 2** is a block diagram representing an example of a device for training a machine learning architecture for generating an adjusted 3D representation of an object according to embodiments of the invention.

As detailed above, in one or several embodiments, the original training set comprises a plurality of 3D CT scans 110 and a plurality of training 2D MR images 130. As represented in Figure 2, the training device 200 for training a machine learning architecture for generating an adjusted 3D representation of an object may comprise a 3D-to-2D pre-processing module 225 to obtain, from each of the 3D CT scans 110, an associated set of 2D CT images (element 120 of Figure 1) corresponding to sections (e.g. cross sections) of the 3D CT scan 110. The training device 200 may also comprise a first ML module 235 comprising the first ML architecture. This first ML module 235 may receive as input training data comprising the plurality of training 2D MR images 130 and the obtained 2D CT images (element 120 of Figure 1, obtained from the 3D-to-2D pre-processing module 225) and output the first learned model 240 adapted to perform a translation from the target imaging modality to the source imaging modality (e.g. MR-to-CT translation). From the plurality of training 2D MR images 130, a respective plurality of 2D pseudo-CT images (element 140 of Figure 1) is obtained through the first ML module 235.

The training device 200 may further comprise a 2D-to-3D post-processing module 245 to obtain, from the obtained 2D pseudo-CT images, corresponding sparse 3D pseudo-CT representations (element 150 of Figure 1).

The training device 200 may further comprise a second ML module 255 comprising the second ML architecture. This second ML module 255 may receive as input training data comprising the sparse 3D pseudo-CT representations (element 150 of Figure 1) and the associated 3D CT scans 110 and output the second learned model adapted to perform the registration between a 3D CT scan and a corresponding 3D pseudo-CT representation. At the end, an operational global trained model 260 is obtained.

**Figure 3** is an example of a flow-chart representing a method for training the first machine learning architecture for generating, from an image compliant with an imaging modality, an image compliant with another imaging modality according to embodiments of the invention.

As represented in Figure 3, the first ML architecture may be trained (step 315) from two training data set: the original training dataset and an induced training dataset. The original training dataset may comprise training 2D MR images 130 and 2D CT images 120 obtained by slicing training 3D CT scans (reference 110 of Figure 1). The induced training dataset may comprise training pairs of images 310 obtained through the first ML architecture, as detailed below with reference to Figure 4.

**Figure 4** represents an example of the first machine learning architecture according to embodiments of the invention. The first ML architecture may comprise a Cycle Generative Adversarial Network (CycleGAN) with two generators $G_{MR}$ and $G_{CT}$ and two discriminators $D_{MR}$ and $D_{CT}$. In embodiments, generators $G_{MR}$ and $G_{CT}$ may be convolutional neural network, for example U-Nets with residual blocks between the contracting and expanding paths for reconstruction stability. Discriminators $D_{MR}$ and $D_{CT}$ may be PatchGAN discriminators. The generators $G_{MR}$ and $G_{CT}$ may be classically associated with respective identity losses $L_{Id}(MR)$ and $L_{Id}(MR)$.

For a given 2D MR image $I_{MR}$ of a set of 2D MR images 130 obtained from a subject, the generator $G_{CT}$ may produce a 2D pseudo-CT image $\hat{I}_{CT} = G_{CT}(I_{MR})$, which may be forwarded into the discriminator $D_{CT}$ along with a "true" 2D CT image $I_{CT}$ randomly sampled from the 3D CT scan associated with the set of 2D MR images 130, i.e. the 3D CT scan of the same subject. A distance between the true 2D CT image $I_{CT}$ and the generated 2D pseudo-CT image $\hat{I}_{CT}$ may be calculated. For example, this distance may be an L1 distance denoted as $L_{adv}(CT)$.

Similarly, for a given 2D CT image $I_{CT}$ of a set of 2D CT images 120 obtained from a subject, the generator $G_{MR}$ may produce a 2D pseudo-MR image $\hat{I}_{MR} = G_{MR}(I_{CT})$, which may be forwarded into the discriminator $D_{MR}$ along with a "true" 2D MR image $I_{MR}$ randomly sampled from the set of 2D MR images 130 obtained from the same subject. A distance between the true 2D MR image $I_{MR}$ and the generated 2D pseudo-MR image $\hat{I}_{MR}$ may be calculated. For example, this distance may be an L1 distance denoted as $L_{adv}(MR)$.

The paths corresponding to the two above paragraphs are represented in solid lines in Figure 4.

To ensure that the generated 2D pseudo-CT image $\hat{I}_{CT}$ is concordant with $I_{MR}$ on a pixel-basis, the generator $G_{MR}$ may be asked to reconstruct $I_{MR}$ from $\hat{I}_{CT}$ by minimizing, for instance, their L1 distance, denoted as $L_{rec}(MR)$. In other words, the generator $G_{MR}$ may produce a 2D pseudo-MR image $\overline{I}_{MR}$ from the 2D pseudo-CT image $\hat{I}_{CT}$ generated by $G_{CT}$, and the distance $L_{rec}(MR)$ between the true 2D MR image $I_{MR}$ and the 2D pseudo-MR image $\overline{I}_{MR}$ may be determined.

Similarly, to ensure that the generated 2D pseudo-MR image $\hat{I}_{MR}$ is concordant with $I_{CT}$ on a pixel-basis, the generator $G_{CT}$ may be asked to reconstruct $I_{CT}$ from $\hat{I}_{MR}$ by minimizing, for instance, their L1 distance, denoted as $L_{rec}(CT)$. In other words, the generator $G_{CT}$ may produce a 2D pseudo-CT image $\overline{I}_{CT}$ from the 2D pseudo-MR image $\hat{I}_{MR}$ generated by $G_{MR}$, and the distance $L_{rec}(CT)$ between the true 2D CT image $I_{CT}$ and the 2D pseudo-CT image $\overline{I}_{CT}$ may be determined.

The paths corresponding to the two above paragraphs are represented in dashed lines in Figure 4.

In embodiments, the first ML architecture may also take advantage of the structure-consistency Modality Independent Neighbourhood Descriptor (MIND) loss described in the article of Heinrich et al.: "MIND: Modality independent neighbourhood descriptor for multi-modal deformable registration", Medical Image Analysis, 16(7): 1423-1435, October 2012.

This loss, denoted as $L_{MIND}$ assesses structural similarity around each voxel v regardless of the intensity distribution. By comparing $I_{MR}$ and $\hat{I}_{CT} = G_{CT}(I_{MR})$, it helps in both transferring style between modalities and enforcing tissue consistency. Such loss for generation of 2D pseudo-CT images may be formulated as:

$$L_{MIND-CT} = E_{mr \sim p(mr)} \left[ \frac{1}{N} \sum_v \left\| MIND_v(G_{CT}(I_{MR})) - MIND_v(I_{MR}) \right\|_1 \right]$$

Similarly, a loss $L_{MIND-CT}$ may be applied for generation of 2D pseudo-MR images:

$$L_{MIND-MR} = E_{ct \sim p(ct)} \left[ \frac{1}{N} \sum_{v} \left\| MIND_v(G_{MR}(I_{CT})) - MIND_v(I_{CT}) \right\|_1 \right]$$

The total loss of the first ML architecture may therefore be:

$$L_{trans} = L_{adv} + \lambda_{rec} L_{rec} + \lambda_{Id} L_{Id} + \lambda_{MIND} L_{MIND}$$

where $\lambda_{rec}$, $\lambda_{Id}$ and $\lambda_{MIND}$ are weights balancing each loss.

As detailed above, each 2D pseudo-CT image $\bar{I}_{CT}$ is generated by $G_{CT}$ from a respective 2D pseudo-MR image $\hat{I}_{MR}$ which is itself generated by $G_{MR}$ from a respective "true" 2D CT image $I_{CT}$. Therefore, from the first ML architecture of Figure 4, pairs of corresponding CT images are obtained, each pair comprising a "true" 2D CT image $I_{CT}$ and a corresponding 2D pseudo-CT image $\bar{I}_{CT}$. Similarly, each 2D pseudo-MR image $\bar{I}_{MR}$ is generated by $G_{MR}$ from a respective 2D pseudo-CT image $\hat{I}_{CT}$ which is itself generated by $G_{CT}$ from a respective "true" 2D MR image $I_{MR}$. Therefore, from the first ML architecture of Figure 4, pairs of corresponding MR images are obtained, each pair comprising a "true" 2D MR image $I_{MR}$ and a corresponding 2D pseudo-MR image $\bar{I}_{MR}$.

These corresponding pairs of MR and/or CT images (reference 310 of Figure 3) may be used as "induced training dataset" for training the first ML architecture, wherein each "true" 2D MR (resp. CT) image $I_{MR}$ (resp. $I_{CT}$) may be seen as the "truth" to which each corresponding 2D pseudo-MR (resp. CT) image $\bar{I}_{MR}$ (resp. $\bar{I}_{CT}$) may be compared.

Referring again to Figure 3, the first ML architecture can be trained from the original training dataset comprising training 2D MR images 130 and 2D CT images 120, and from the induced training dataset comprising corresponding pairs 310 of MR and/or CT images, to obtain a first trained model 240 adapted to perform a translation from one imaging modality to another (e.g., MR-to-CT translation).

**Figure 5** represents an example of the second machine learning architecture for performing registration between two 3D representations according to embodiments of the invention.

An aim of the second machine learning architecture is to allows a mapping between the 3D CT scan of a subject onto the generated sparse 3D pseudo-CT scan. It is noted that the 3D CT scan and the sparse 3D pseudo-CT scan have the same size (typically, they are represented by respective matrices of pixels having the same size). In the following, the 3D CT scan (element 110 of Figure 1) is denoted $m$ and the generated sparse 3D pseudo-CT scan (element 150 of Figure 1) is denoted $f$.

The second ML architecture may typically comprise a network that produces, for each voxel of the 3D CT scan, a 3D displacement field (or "deformation field") $R_{f,m}$ to be applied to said voxel of the 3D CT scan to match the 3D CT scan with the generated sparse 3D pseudo-CT scan. This 3D displacement field may comprise, for each voxel $v$, a respective triplet of values, each value representing a respective component in a 3D coordinate system.

The second ML architecture may comprise a Convolutional Neural Network (CNN) trained with pairs of 3D volumes, each pair comprising a 3D CT scan and a corresponding sparse 3D pseudo-CT scan. CNNs are well-known tools for the person skilled in the art to perform registration, therefore the architecture of the CNN if not further detailed in this specification.

The CNN may output the displacement field, which may comprise two components: an affine transformation matrix 510 and a deformable transformation matrix 520.

The affine transformation matrix 510 represents a linear transformation to apply to the 3D CT scan (for example so that a region of interest of the 3D CT scan is globally at the same "place" as the same region of interest in the sparse 3D pseudo-CT scan). Such linear transformation may be decomposed into three basic transformations: rotation, dilation and translation, which may be represented in 3D by 12 parameters. As represented in Figure 5, the affine transformation matrix 510 may therefore be a matrix with 3 lines and 4 rows, the 3 first rows (i.e., 9 parameters) may represent the rotation and the dilation, and the last row (3 parameters) may represent the translation.

The deformable transformation matrix 520 may represent the non-rigid transformation to apply to each voxel of the 3D CT scan. It may be a matrix of the same size as the 3D CT scan and the sparse 3D pseudo-CT scan, each element of the matrix comprising 3 values to respectively apply to each component of the concerned voxel.

The final 3D displacement field $R_{f,m}$ to be applied to said voxel of the 3D CT scan is therefore obtained from the affine transformation matrix 510 and the deformable transformation matrix 520.

Advantageously, the training of the second ML architecture is fully unsupervised. This training may be performed by maximizing the similarity between $f$ and $R_{f,m}(m)$.

Since $f$ is sparse (i.e., it contains sections filled with zero values), a classical volumetric loss is not optimal as it would induce noise within the gradient of pixels registered onto empty slices by imposing such pixels to be equal to 0. To circumvent this issue, it is possible to mask the pixel-wise loss for empty slices as follows:

$$L_{sim}^{2D}\left(f, R_{f,m}(m)\right) = \sum_{i; f_i \neq 0} \left\| f_i - R_{f,m}(m)_i \right\|_2$$

wherein $f_i$ designates a section (i.e., a slice) of f and $\|.\|_2$ designates the Euclidean distance. In other words, the loss is computed by computing the mean square error (MSE) only on non-zero sections of $f$. Other embodiments are possible. For example, the Normalized Cross-Correlation (NCC) loss may be used instead of the MSE.

In addition, because the loss is masked for CT slices within the original sparse 3D pseudo-CT scan, there may be additional degrees of freedom for the registration. To bypass this issue, it is possible, in one or several embodiments, to add a 3D regularization loss $L_{regu}^{3D}$ that penalizes large local variations of the displacement field by minimizing the mean square error of the spatial gradient of $R_{f,m}$ as proposed in the article of Balakrishnan et al., "VoxelMorph: A Learning Framework for Deformable Medical Image Registration", IEEE Transactions on Medical Imaging, 38(8):1788- 1800, August 2019.

Together, $L_{sim}^{2D}$ and $L_{regu}^{3D}$ make it possible to obtain a final transformation precise, smooth and with realistic mapping, while enforcing the continuity of inter-slice displacements with respect to the surrounding non-empty ones. The total loss for the second ML architecture may therefore be formulated as follows:

$$L_{reg}(f, m, R) = \lambda_{sim} L_{sim}^{2D}\left(f, R_{f,m}(m)\right) + \lambda_{regu}^{3D}\left(R_{f,m}\right)$$

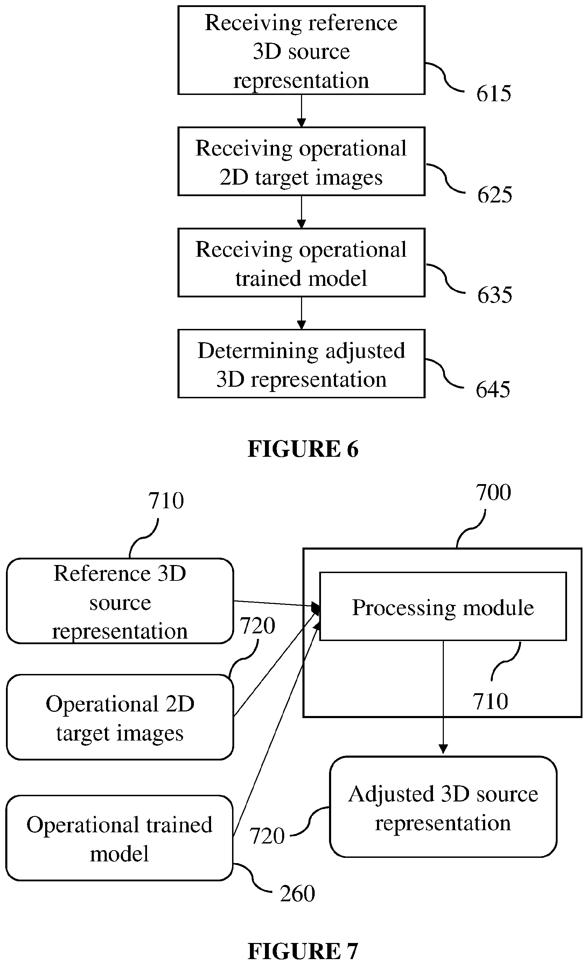

**Figure 6** is an example of a flow-chart representing a method for generating an adjusted 3D representation of an object according to embodiments of the invention.

Once the operational global trained model 260 is obtained (i.e., after the training phase represented in Figure 1), this trained model 260 may be applied to obtain, from the 3D CT scan and a plurality of 2R MR images of a subject, a deformed 3D CT scan adjusted to the plurality of 2R MR images (prediction phase).

In one or several embodiments, a 3D CT scan (also referred to as "reference 3D source representation") of an area of a subject body may be received at step 615. A plurality of 2R MR images (also referred to as "operational 2D target images") of at least part of the same area of the same subject body may be received at step 625. Also, operational (global) trained model 260 may be received at step 635. It is noted that steps 615, 625 and 635 may be performed in parallel or successively, in any order.

The 3D CT scan and the plurality of 2D MR images are then used as inputs of the operational trained model to determine, at step 645, a deformed 3D CT scan adjusted to the plurality of 2R MR images.

**Figure 7** is a block diagram representing an example of a device 700 for generating an adjusted 3D representation of an object according to embodiments of the invention.

The device 700 may comprise an input interface for receiving the 3D CT scan 710 of the area of the subject body, the plurality of 2D MR images 720 of at least part of the same area of the same subject body, and the operational trained model 260. The device 700 may also comprise a processing module 710 for determining, by using the 3D CT scan 710 and the plurality of 2D MR images 720 as inputs of the operational trained model 260, a deformed 3D CT scan 720 adjusted to the plurality of 2R MR images.

**Figure 8** illustrates a device for training a machine learning architecture for generating an adjusted 3D representation of an object and/or for generating an adjusted 3D representation of an object according to embodiments of the invention.

In these embodiments, the device 800 comprises a computer, this computer comprising a memory 801 to store program instructions loadable into a circuit and adapted to cause a circuit 802 to carry out steps of the methods of **Figures 1** and **6** when the program instructions are run by the circuit 802. The memory 801 may also store data and useful information for carrying steps of the present invention as described above.

The circuit 802 may be for instance:

- a processor or a processing unit adapted to interpret instructions in a computer language, the processor or the processing unit may comprise, may be associated with or be attached to a memory comprising the instructions, or

- the association of a processor / processing unit and a memory, the processor or the processing unit adapted to interpret instructions in a computer language, the memory comprising said instructions, or

- an electronic card wherein the steps of the invention are described within silicon, or

- a programmable electronic chip such as a FPGA chip (for « Field-Programmable Gate Array »).

The computer may also comprise an input interface 803 for the reception of training data and/or the operational data of a subject and an output interface 804 to provide the trained operational model and/or the deformed 3D CT scan of a subject.

To ease the interaction with the computer, a screen 805 and a keyboard 806 may be provided and connected to the computer circuit 802.

Furthermore, the flow charts represented in **Figures 1** and **6** can represent all or part of the steps of a program which may be executed by a processor. As such, **Figures 1** and **6** may correspond to flow charts of the general algorithm of computer programs within the meaning of the invention.

Experiments and results

Performances of the method for determining an adjusted 3D representation of an object from a plurality of 2D images of at least part of said object have been assessed with pelvis 3D CT scans and 2D MR images. More precisely, two private clinical datasets for patients undergoing radiotherapy (RT) have been used. The first dataset comprises 451 pairs between the planning CT and the 0,35T TrueFISP MR sequences of treatment delivery. The second dataset involves 217 pairs between the planning CT and the 1,5T T2 MR sequences. 0,35T TrueFISP MR and 1,5T T2 MR correspond to different image qualities (resolution of 0,35T TrueFISP MR being lower than resolution of 1,5T T2 MR) Such a gap in texture resolution is an argument for the ability of the method to perform in many study cases. The ratio between the 3D planning CT and the multi-slice treatment MR in terms of slices was 10 : 1 (i.e. the MR images corresponds in average to 1 slice out 10 slices of the 3D CT).

Both datasets were preprocessed independently with normalization, resampling and cropping to get $256 \times 256 \times 96$ (x, y, z) volumes with an (x, y) resolution of $1mm^2$ and a z resolution of 3mm. For each volume and modality, 8 anatomical structures were manually segmented by internal experts: anal canal, bladder, left/right femoral head, rectum, penile bulb, seminal vesicle and prostate - when applicable. Each dataset was separated into three groups for training (60%), validation (20%) and testing (20%). Loss weights were set as follows to have balanced leverage between each component: $\lambda_{rec} = \lambda_{Id} = 10$, $\lambda_{MIND} = \lambda_{sim} = 5$ and $\lambda_{regu} = 0.1$.

Performance of the MT-to-CT translation part of the method has been assessed against two prior art methods, namely conventional CycleGAN and CycleGAN with a MIND compensation in the loss, by using two metrics quantifying the quality of image reconstruction: SSIM (for "Structural SIMilarity"), which assesses the structural degradation of a reconstructed image compared to the original one, and FID (for "Fréchet inception distance"), which measures the distance between distributions of generated and original sets as latent vectors. Also, results were compared between the new method without MIND compensation and with MIND compensation.

The Results for MT-to-CT translation part are provided below:

| Method | 0.35T TrueFISP $\rightarrow$ 3D CT | | 1.5T T2 $\rightarrow$ 3D CT | |
|---|---|---|---|---|
| | SSIM | FID | SSIM | FID |
| CycleGAN | $0.751 \pm 0.03$ | 132.5 | $0.763 \pm 0.02$ | 124.4 |
| CycleGAN + MIND | $0.768 \pm 0.08$ | 112.1 | $0.769 \pm 0.02$ | 116.8 |
| New method (no MIND) | $0.763 \pm 0.01$ | 112.9 | $0.768 \pm 0.04$ | 119.1 |
| New method (with MIND) | $0.789 \pm 0.06$ | 89.8 | $0.795 \pm 0.05$ | 80.4 |

As shown in the above table, performance of the new method with MIND compensation outperforms the CycleGAN algorithms, with or without MIND. Performance of the new method without MIND compensation is similar than CycleGAN with MIND compensation. These results suggest that the joint learning of both the first (translation) ML architecture and the second (registration) ML architecture improves performance of each architecture considered independently from the other. In other words, the concurrent registration module improves the translation module by giving feedback on reconstruction error though backpropagation.

Performance of registration part of the new method has been assessed against several methods of the prior art (SyN (ANTs) affine, SyN deformable, VoxelMorph (SSIM), VoxelMorph (MIND)), by using two metrics quantifying the quality of image segmentation: Dice score and Hausdorff distance between masks of 2D MR images and masks of the deformed 3D CT representation. The Hausdorff distance is a real positive or null value which represents the highest of all the distances from a point in one set to the closest point in the other set, and is minimized (i.e., close to 0) when registration is successful. Dice score is a real value comprised between 0 and 1 which measures the level of intersection between the two segmentation masks, and is maximized (i.e., close to 1) when registration is successful.

Also, results were compared between different embodiments of the registration method: when only affine transformation is considered for the displacement field (denoted "affine" in the second table below), without considering MIND compensation (denoted "no MIND" in the second table below), without the first ML architecture (denoted "no end-to-end" in the second table below) - i.e., when the second ML architecture is trained independently from the first ML architecture. Finally, results were compared between the method with NCC-based second loss and the method with MSEbased second loss.

Results are provided in the second table below:

| Method | 0.35T TrueFISP → 3D CT | | 1.5T T2 → 3D CT | |
|---|---|---|---|---|
| | Dice | Hausdorff | Dice | Hausdorff |
| SyN (ANTs) affine | 69.9 ± 1.7 | 12.05 ± 0.09 | 68.7 ± 0.9 | 13.49 ± 0.10 |
| SyN deformable | 75.2 ± 1.2 | 9.72 ± 0.13 | 76.1 ± 1.1 | 9.19 ± 0.11 |
| VoxelMorph (SSIM) | 81.2 ± 1.6 | 7.96 ± 0.10 | 80.9 ± 0.9 | 7.91 ± 0.08 |
| VoxelMorph (MIND) | 81.5 ± 1.5 | 7.82 ± 0.07 | 81.3 ± 1.5 | 7.88 ± 0.14 |
| Affine | 70.5 ± 1.2 | 11.22 ± 0.09 | 69.4 ± 0.8 | 11.94 ± 0.14 |
| No MIND | 79.8 ± 1.1 | 8.86 ± 0.08 | 80.8 ± 1.2 | 7.88 ± 0.06 |
| No end-to-end | 81.2 ± 1.2 | 8.01 ± 0.08 | 81.4 ± 0.9 | 7.62 ± 0.12 |
| NCC | 84.6 ± 0.9 | 7.25 ± 0.05 | 85.3 ± 1.4 | 6.24 ± 0.09 |
| MSE | 83.8 ± 1.2 | 7.48 ± 0.13 | 86.1 ± 1.0 | 5.84 ± 0.15 |

The end-to-end approach reaches the best performance among all other approaches, which confirms that the concurrent approach (i.e. the joint training of the two ML architectures) outperforms independent trainings.

A person skilled in the art will readily appreciate that various parameters disclosed in the description may be modified and that various embodiments disclosed may be combined without departing from the scope of the invention. Of course, the present invention is not limited to the embodiments described above as examples. It extends to other variants. For example, the first and/or the second trained models may be used independently from each other. For instance, the first trained model may be used for performing an image translation from a first imaging modality to another modality, and the second trained model may be used for performing registration between any two 3D volumes. Also, even if the second trained model has been presented above in the context of CT images (i.e., the "source imaging modality"), it can be adapted to perform registration between MR volumes (i.e. the "target imaging modality"). Also, even if the present invention has been more specifically described in the context of medical imaging, it may be applied to any imaging field, for example in geophotography or in astrophotography.

**Claims**

1. A method implemented by computer for training a machine learning architecture for generating an adjusted 3D representation of an object based on a reference 3D representation of said object compliant with a source imaging modality and a plurality of images of at least part of said object compliant with a target imaging modality, so that 2D sections of the adjusted 3D representation are at least partially registered with images of the plurality of images, the machine learning architecture comprising a first machine learning architecture and a second machine learning architecture, the method comprising :

- receiving training target images compliant with the target imaging modality and 3D training source representations compliant with the source imaging modality;
- obtaining training source images corresponding to 2D sections of the received 3D training source representations; and
- jointly training the first machine learning architecture and the second machine learning architecture to obtain:

  o a first learned model associated with the first machine learning architecture, said first learned model being adapted to generate, from an input target image compliant with the target imaging modality, a simulated source image compliant with the source imaging modality respectively associated with the input target image; and
  o a second learned model associated with the second machine learning architecture, said second learned model being adapted to generate, from a sparse 3D representation compliant with the source imaging modality and an original 3D representation compliant with the source imaging modality, a deformation field to be applied to voxels of the original 3D representation so as to obtain an adjusted 3D representation whose 2D sections are at least partially registered with 2D sections of the sparse 3D representation;

  wherein the first machine learning architecture is trained based on first set of training data comprising the training target images and the training source images;
  wherein the second machine learning architecture is trained based on a second set of training data comprising the received 3D training source representations and sparse 3D representations compliant with the source imaging modality, said sparse 3D representations being obtained by:

  - obtaining, from at least part of the training target images, simulated source images compliant with the source imaging modality through the first machine learning architecture;
  - constructing the sparse 3D representations so that sections of said sparse 3D representations correspond to said simulated source images.

2. The method of any one of claim **1,** wherein the first machine learning architecture is associated with a first loss and the second machine learning architecture is associated with a second loss, wherein the joint training of the first machine learning architecture and the second machine learning architecture comprises a joint minimization of the first loss and the second loss.

3. The method of claim **1** or **2,** wherein the first machine learning architecture and/or the second machine learning architecture are trained in an unsupervised manner.

4. The method of any one of the preceding claims, wherein each of the 3D training source representations represents a part of a body of a respective subject among a plurality of subjects, wherein the training target images comprise sets of training target images, each set of training target images being associated with a respective subject of the plurality of subjects and representing successive sections of the part of the body of said respective subject.

5. The method of any one of the preceding claims, wherein the first machine learning architecture is adapted to simulate, from an image compliant with one imaging modality among the source imaging modality and the target imaging modality, a corresponding image compliant with the other imaging modality, wherein the first machine learning architecture is further trained based on pairs of images, each pair of images comprising:

  - a training image compliant with one imaging modality among the target imaging modality and the source imaging modality, said training image being one of the training source images and the training target images; and
  - a corresponding new image compliant with the same imaging modality and obtained from the training image of the pair through the first machine learning architecture.

6. The method of claim 5, wherein, for each pair of images, the new image is obtained by:

  - obtaining, from the training image of said pair, a corresponding intermediate image compliant with the other imaging modality through the first machine learning architecture;
  - obtaining, from the obtained intermediate image, the new image through the first machine learning architecture.

7. The method of claim 5 or 6, in combination with claim 2, wherein the first loss is function of:

- a first term representative of a difference between a training image among the training source images and the training target images, said training image being compliant with one imaging modality among the target imaging modality and the source imaging modality, and a corresponding image compliant with the other imaging modality and generated from said training image through the first machine learning architecture; and
- a second term representative of a difference between the images of a pair of images.

8. The method of any one of the preceding claims, in combination with claim 2, wherein the second loss is function of a term representative of differences between sections of the sparse 3D representations that correspond to simulated source images and corresponding sections of adjusted 3D representations respectively generated from the sparse 3D representations through the second machine learning architecture.

9. The method of any one of the preceding claims, wherein the target imaging modality is magnetic resonance imaging and the source imaging modality is computed tomography imaging.

10. The method of any one of the preceding claims, wherein the first machine learning architecture comprises a cycle generative adversarial network, GAN.

11. The method of any one of the preceding claims, wherein the second machine learning architecture comprises a convolutional neural network, CNN.

12. A method implemented by computer for generating an adjusted 3D representation of an object based on a reference 3D source representation of said object compliant with a source imaging modality and a plurality of reference target images of at least part of said object compliant with a target imaging modality, the method comprising:

- obtaining a plurality of simulated source images compliant with the source imaging modality and respectively corresponding to the plurality of reference target images, by applying the first learned model of any one of claims 1 to 11 to reference target images;
- obtaining a sparse 3D source representation whose 2D sections correspond to simulated source images among the plurality of simulated source images;
- determining, by applying the second learned model of any one of claims 1 to 10 to the reference 3D source representation and the sparse 3D source representation, a deformation field to be applied to voxels of the reference 3D source representation so as to at least partially register 2D sections of the reference 3D source representation with corresponding 2D sections of the sparse 3D source representation; and
- obtaining the adjusted 3D representation of the object by applying the determined deformation field to the 3D reference source representation.

13. The method of claim 12, wherein the reference target images represent successive sections of at least part of the object.

14. A device comprising a processor configured to carry out a method according to any one of claims 1 to 13.

15. A computer program product comprising instructions which, when the program is executed by a computer, cause the computer to carry out the method of any one of claims 1 to 13.

FIGURE 1

EP 4 336 460 A1

**FIGURE 2**

**FIGURE 3**

**FIGURE 4**

**FIGURE 8**

FIGURE 5

Receiving reference
3D source
representation

615

Receiving operational
2D target images

625

Receiving operational
trained model

635

Determining adjusted
3D representation

645

**FIGURE 6**

710

700

Reference 3D
source
representation

720

Processing module

Operational 2D
target images

710

Operational trained
model

720

Adjusted 3D source
representation

720

260

**FIGURE 7**

Europäisches
Patentamt

European
Patent Office

Office européen
des brevets

# EUROPEAN SEARCH REPORT

Application Number

EP 22 19 4697

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XU ZHE ET AL: "Adversarial Uni- and Multi-modal Stream Networks for Multimodal Image Registration", 29 September 2020 (2020-09-29), 16TH EUROPEAN CONFERENCE – COMPUTER VISION – ECCV 2020, CORNELL UNIVERSITY LIBRARY, 201 OLIN LIBRARY CORNELL UNIVERSITY ITHACA, NY 14853, PAGE(S) 222 – 232, XP047563907, [retrieved on 2020-09-29] * figures 1,2,3,4,5 * * section 1 "introduction" – paragraph 1 * * section 1 "introduction" – subsection "related work", paragraph 2 * * section 2 "method" – paragraphs 1 and 2 * * section 2 "methods" – first paragraph * * section 2.1 "image-to-image translation with unpaired data" * * section 2.2 "dual-stream multimodal image registration network" – paragraph 2 * * section 2.2 "dual-stream multimodal image registration network" – subsection "network details" * * section 3 "experiments and results" – subsection "dataset and preprocessing" * * section 3.1 "results for ct-to-mr translation" – paragraphs 1 and 2 * * section 3.2 "registration results" – paragraph 2 * * section 3.3 "the effect of each deformation field" – paragraph 1, last sentence * * section 4 "conclusion" * | 1-15 | INV. G06T19/20 |
| | ----- -/-- | | TECHNICAL FIELDS SEARCHED (IPC) G06T |

The present search report has been drawn up for all claims

| Place of search | Date of completion of the search | Examiner |
|---|---|---|
| Berlin | 28 January 2023 | Gauthier, J |

EPO FORM 1503 03.82 (P04C01)

Europäisches Patentamt

European Patent Office

Office européen des brevets

# EUROPEAN SEARCH REPORT

Application Number

EP 22 19 4697

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YANG HERAN ET AL: "Unpaired Brain MR-to-CT Synthesis Using a Structure-Constrained CycleGAN", 20 September 2018 (2018-09-20), 16TH EUROPEAN CONFERENCE - COMPUTER VISION - ECCV 2020, CORNELL UNIVERSITY LIBRARY, 201 OLIN LIBRARY CORNELL UNIVERSITY ITHACA, NY 14853, PAGE(S) 174 - 182, XP047557315, [retrieved on 2018-09-20] * figures 1,2,3,5 * * section 1 "introduction" * * section 2 "method" * | 1-15 | |
| A | BALAKRISHNAN GUHA ET AL: "VoxelMorph: A Learning Framework for Deformable Medical Image Registration", IEEE TRANSACTIONS ON MEDICAL IMAGING, IEEE, USA, vol. 38, no. 8, 1 August 2019 (2019-08-01), pages 1788-1800, XP011736963, ISSN: 0278-0062, DOI: 10.1109/TMI.2019.2897538 [retrieved on 2019-07-30] * figures 2,3,6 * * section I "introduction" * * section IV "method" * | 1-15 | TECHNICAL FIELDS SEARCHED (IPC) |

-/--

The present search report has been drawn up for all claims

| Place of search | Date of completion of the search | Examiner |
|---|---|---|
| Berlin | 28 January 2023 | Gauthier, J |

EPO FORM 1503 03.82 (P04C01)

Europäisches Patentamt
European Patent Office
Office européen des brevets

**EUROPEAN SEARCH REPORT**

Application Number

EP 22 19 4697

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZEKANG CHEN ET AL: "Unsupervised Multi-Modal Medical Image Registration via Discriminator-Free Image-to-Image Translation", ARXIV.ORG, CORNELL UNIVERSITY LIBRARY, 201 OLIN LIBRARY CORNELL UNIVERSITY ITHACA, NY 14853, 28 April 2022 (2022-04-28), XP091210219, * figures 1-4 * * section 3 "method", paragraphs 1 and 2 * * section 3.1 "network" * * section 3.2 "discriminator-free translation network" * * section 3.4 "final objective" * | 1-15 | |
| A | MATHIAS UNBERATH ET AL: "The Impact of Machine Learning on 2D/3D Registration for Image-guided Interventions: A Systematic Review and Perspective", ARXIV.ORG, CORNELL UNIVERSITY LIBRARY, 201 OLIN LIBRARY CORNELL UNIVERSITY ITHACA, NY 14853, 4 August 2021 (2021-08-04), XP091030510, * the whole document * | 1-15 | |
| X,P | LEROY AMAURY ET AL: "End-to-End Multi-Slice-to-Volume Concurrent Registration and Multimodal Generation", 17 September 2022 (2022-09-17), In Medical Image Computing and Computer Assisted Intervention – MICCAI 2022: 25th International Conference, Singapore, September 18-22, 2022, Proceedings, Part VI (pp. 152-162). Cham: Springer Nature Switzerland., XP047633809, ISSN: 0302-9743 ISBN: 978-3-030-41298-2 [retrieved on 2022-09-17] * the whole document * | 1-15 | TECHNICAL FIELDS SEARCHED (IPC) |

The present search report has been drawn up for all claims

| Place of search | Date of completion of the search | Examiner |
|---|---|---|
| Berlin | 28 January 2023 | Gauthier, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

**Non-patent literature cited in the description**

- **HEINRICH et al.** MIND: Modality independent neighbourhood descriptor for multi-modal deformable registration. *Medical Image Analysis,* October 2012, vol. 16 (7), 1423-1435

- **BALAKRISHNAN et al.** VoxelMorph: A Learning Framework for Deformable Medical Image Registration. *IEEE Transactions on Medical Imaging,* August 2019, vol. 38 (8), 1788-1800